# EUROPÄISCHE PATENTSCHRIFT

(11) **EP 2 430 395 B1**
(45) Veröffentlichungstag und Bekanntmachung des Hinweises auf die Patenterteilung: **24.07.2013**
(21) Anmeldenummer: 10720412.5
(22) Anmeldetag: 17.05.2010
(51) Int. Cl.: G01B 11/24, G02B 21/00, A61B 5/00

(54) **VERFAHREN SOWIE MESSANORDNUNG ZUM DREIDIMENSIONALEN MESSEN EINES OBJEKTES**
METHOD AND MEASURING ARRANGEMENT FOR THE THREE-DIMENSIONAL MEASUREMENT OF AN OBJECT
PROCÉDÉ ET DISPOSITIF DE MESURE TRIDIMENSIONNELLE D'UN OBJET

(30) Priorität: 15.05.2009 DE 102009025815
(43) Veröffentlichungstag der Anmeldung: 21.03.2012
(73) Patentinhaber: DeguDent GmbH, 63457 Hanau (DE)
(72) Erfinder: STOCK, Karl, 73479 Ellwangen (DE); ZINT, Michael, 89075 Ulm (DE); GRASER, Rainer, 89073 Ulm (DE); HIBST, Raimund, 89155 Erbach (DE)
(74) Vertreter: Stoffregen, Hans-Herbert
(86) Internationale Anmeldenummer: PCT/EP2010/056755
(87) Internationale Veröffentlichungsnummer: WO 2010/130843

(56) Entgegenhaltungen:
- WO-A1-2005/031435
- DE-A1-102005 052 743
- DE-A1-102007 019 267
- US-A1- 2002 027 708

## Beschreibung

Die Erfindung bezieht sich auf ein Verfahren zum Messen der Form zumindest eines Abschnitts eines Objekts, insbesondere eines semitransparenten Objekts wie zumindest Abschnitts eines Zahns, unter Verwendung zumindest einer Lichtquelle zur Erzeugung von Licht mit vorzugsweise einem breitbandigen Spektrum, einer Einrichtung zur Erzeugung eines multifokalen Beleuchtungsmusters, eines Objektivs mit großer chromatischer Aberration zur Abbildung von Foki des Beleuchtungsmusters auf das Objekt, einer Detektiereinrichtung zur Ermittlung der Wellenlängenspektren der konfokal über das Objektiv auf das Objekt abgebildeten Foki, wobei aus dem jeweiligen Wellenlängenspektrum spektrale Peaklage eines jeden Fokus bestimmt wird, aus der die Erstreckung des Objekts in Richtung des Abbildungsstrahls (Z-Koordinate) berechnet wird, das multifokale Beleuchtungsmuster über zwischen der Lichtquelle und dem Objektiv großer chromatischer Aberration angeordnete Lichtleiter erzeugt wird, wobei das Objektiv objektseitige Enden der Lichtleiter auf das Objekt und von dem Objekt remittiertes Licht auf die objektseitigen Enden der Lichtleiter abbildet und durch die Lichtleiter geleitetes remittiertes Licht auf die Detektiereinrichtung gelenkt wird, wobei eine unterschiedliche objektseitige Anordnung der Enden der Lichtleiter und beleuchtungs- bzw. detektionsseitige Anordnung der Enden der Lichtleiter erzeugt wird, damit eine objektseitige Messpunkteverteilung von einer beleuchtungs- bzw. detektionsseitigen Mikrolinsen- bzw. Pinholeverteilung unabhängig ist.

Ferner nimmt die Erfindung Bezug auf eine Messanordnung zum dreidimensionalen Messen von zumindest einem Teil eines Objekts, insbesondere eines Semitransparenten Objekts wie Zahns oder Abschnitts eines solchen, umfassend zumindest eine Lichtquelle mit einem kontinuierlichen, insbesondere breitbandigen Spektrum, eine Einrichtung zur Erzeugung eines multifokalen Beleuchtungsmusters, ein Objektiv mit großer chromatischer Abberation zur Abbildung von Foki des Beleuchtungsmusters auf das Objekt, eine Detektiereinheit wie Kamera-Chip zur Ermittlung der Wellenlängenspektren der über das Objektiv auf das Objekt abgebildeten Foki sowie eine spektraldispersive Einrichtung, auf die von dem Objekt remittiertes Licht über das Objektiv abbildbar ist, wobei zwischen der Lichtquelle und dem Objektiv das Beleuchtungsmuster erzeugende Lichtleiter angeordnet sind, deren objektseitige Enden in einer Abbildungsebene oder einen Abbildungsebenen umfassenden Bereich des Objektivs angeordnet sind, und zwischen den beleuchtungsseitigen Enden der Lichtleiter und der Detektiereinrichtung eine Umlenkeinrichtung für das aus dem Lichtleiter austretende und von dem Objekt remittierte Licht angeordnet ist, wobei die objektseitigen Enden der Lichtleiter derart angeordnet sind, dass objektseitig eine Messpunkteverteilung abbildbar ist, die von einer beleuchtungs- bzw. detektionsseitigen Mikrolinsen- bzw. Pinholeverteilung unabhängig ist.

In vielen Bereichen der Technik stellt sich die Aufgabe, die dreidimensionale Struktur von Körpern zu messen. Beispielhaft sei hier die Bestimmung der Zahnform genannt, die zur Anfertigung von Zahnersatz notwendig ist. Hier sind vor allem Verfahren vorteilhaft, die das Anfertigen eines Gipsabdrucks erübrigen. In der Literatur sind eine Reihe von Verfahren zur Erfassung der dreidimensionalen Struktur von Körpern beschrieben. Bei den optischen Verfahren sind unter anderem die Streifenprojektionsverfahren bzw. Phasenschiftverfahren, die optische Kohärenztomographie und die Holographie zu nennen. Ein zur intraoralen Zahnform-Erfassung käufliches System basiert beispielsweise auf dem Phasenschiftverfahren.

Gerade auf wenig kooperativen Körpern, wie sie beispielsweise der Zahn aufgrund seiner starken Volumenstreuung darstellt, versagen die genannten Verfahren vielfach. Bei den Streifenprojektionsverfahren führt beispielsweise die Streuung zu einer Unschärfe der Streifen und damit zu einer verminderten Auflösung.

Alternativ wird zur Abbildung des Fokus bzw. der Foki einer breitbandigen Lichtquelle eine geeignete Optik mit stark wellenlängenabhängiger Brennweite verwendet. Dadurch werden die Foki abhängig von der Wellenlänge in unterschiedlichen Abständen zum Objektiv scharf abgebildet. Nach rückwärtiger Abbildung der Foki über das Objektiv in das Pinhole bzw. Pinhole-Array ist für die Wellenlänge, die bei diesem Probenabstand scharf abgebildet wird, ein Intensitätsmaximum detektierbar. Durch Bestimmung der spektralen Peaklage lässt sich dann der Abstand der Probe zum Objektiv in diesem Punkt und damit letztendlich die dreidimensionale Struktur des Körpers bestimmen. Diese Auswertung erfolgt entweder punktweise über ein Spektrometer oder linienweise über ein Linienspektrometer mit Kamera-Chip. Gerade die Multifoki-Anordnung, vorzugsweise mit Mikrolinsen-Array und angepasstem Pinhole-Array, ist wegen des geringen zu erwartenden Zeitbedarfs für die Bildaufnahme besonders vielversprechend.

In der DE-A-10 2006 007 172, WO-A-2007/090865 und DE-A-10 2007 019 267 sind unterschiedliche Anordnungen nach diesem Prinzip beschrieben. Allen gemeinsam ist, dass alle Komponenten in einem Gerät integriert sind. Lediglich in der DE-A-10 2007 019 267 wird eine mögliche Zufuhr der Lichtquelle über Lichtleiter beschrieben. Gerade bei Messungen am Zahn, wo einerseits der zur Verfügung stehende (Mund-)Raum begrenzt ist und der Zahnarzt das Gerät manuell führen muss, ist ein möglichst kompaktes und leichtes Handstück mit möglichst wenigen Komponenten wünschenswert. Ein weiterer Nachteil der beschriebenen Anordnungen ist die feste Zuordnung des Mikrolinsen- und Pinhole-Musters auf der einen Seite und der Messpunkteverteilung probenseitig, was sowohl ein Kompromiss für die bestmögliche Messpunkteverteilung auf der Probe als auch für die Spektrenverteilung auf dem Kamera-Chip darstellt.

Aus der EP-B-0 321 529 ist eine Messanordnung zur Messung der Abstände zwischen einem Objektiv mit großer chromatischer Aberration und einem Objekt bekannt. Zur Detektion wird eine Schwarz-Weiß-CCD-Flächenkamera benutzt, der eine spektraldispersive Apparatur vorgeordnet ist, die einen Eingangsspalt aufweist. Hierdurch wird die Wellenlängeninformation für jeden Punkt in eine Ortsinformation umgesetzt, um ein Profilbild der Oberfläche des Objekts zu gewinnen.

Die EP-B-0 466 979 bezieht sich auf eine Anordnung zur simultanen konfokalen Bilderzeugung. Hierzu werden über eine Lochrasterblende wie Nipkow-Scheibe Lichtpunkte erzeugt, die fokussiert auf ein Objekt abgebildet werden. Als Detektiereinheit wird eine CCD-Array-Kamera benutzt.

Aus der DE-A-102 42 374 ist ein konfokaler Abstandssensor mit einer Abbildungsoptik mit chromatischer Aberration bekannt, der für die Inspektion im Elektronikbereich bestimmt ist. Als Lichtquelle kann eine solche mit einer Mehrzahl von Punktlichtquellen benutzt werden. Als Lichtempfänger gelangen Punktdetektoren zum Einsatz, wobei jeweils ein Punktdetektor und eine Punktlichtquelle einander zugeordnet und konfokal zueinander angeordnet sind.

Aus der DE-A-103 21 885 ist eine konfokale Messanordnung zum dreidimensionalen Messen eines Objekts mit chromatischer Tiefenaufspaltung bekannt, bei der mittels eines Mikrolinsen-Arrays eine Vielzahl von Foki erzeugt und auf das Objekt abgebildet werden. Das reflektierte Licht wird in die Ebene der Mikrolinsenfoki zurückfokussiert. Mit der Anordnung werden zwei- oder dreidimensionale Mikroprofile von Messobjekten oder zwei- oder dreidimensionale Transparenz- oder Reflexionsprofile gemessen.

Aus der DE-A-10 2005 052 743 ist ein Messsystem zur Vermessung von Grenz- oder Oberflächen von Werkstücken bekannt. Dabei wird mittels eines Lichtleitfaserbündels über ein eine große chromatische Aberration aufweisendes Objektiv ein Beleuchtungsmuster auf ein zu messendes Objekt abgebildet. Die von dem Objekt remittierte Strahlung wird sodann über die Lichtleitfasern und einen Strahlteilerwürfel auf einen Spektrografen geleitet. Dabei kann die remittierte Strahlung ein Beugungsgitter durchsetzen, um das Licht in einzelne Wellenlängen zu zerlegen. Die verwendeten Lichtleitfasern können ferner gegeneinander verdreht sein.

Gegenstand der WO-A-2005/031435 ist ein konfokales Endomikroskop, wobei über eine Mikrolinse Licht auf Lichtleitern abgebildet wird.

Ein Konfokal-Mikroskop ist aus der US-A-2002/0027708 bekannt

Der vorliegenden Erfindung liegt die Aufgabe zugrunde, eine Messanordnung und ein Verfahren der eingangs genannten Art so weiterzubilden, dass mit konstruktiv einfachen Maßnahmen hochpräzise gemessen werden kann, wobei sowohl eine bestmögliche Messpunkteverteilung auf der Probe als auch eine optimale Spektrumverteilung in der Detektiereinrichtung ermöglicht werden soll. Auch soll zum Messen, insbesondere bei einem Intraoralmessen, eine kompakte Anordnung zur Verfügung gestellt werden, die ein einfaches Handhaben ermöglicht.

Zur Lösung der Aufgabe sieht eine Messanordnung der eingangs genannten Art vor, dass ein Teil der Lichtleiter unbeleuchtet ist und dass zur Untergrundmessungt damit probenseitig nur das remittierte Licht des Untergrundanteils erfasst wird, das über den Teil der unbeleuchteten Lichtleiter auf die Detektiereinheit oder einen gesonderten Kamera-Chip abbildbar ist.

Verfahrensmäßig zeichnet sich die Erfindung grundsätzlich dadurch aus, dass ein Teil der Lichtleiter unbeleuchtet bleibt und dass über den unbeleuchteten Teil der Lichtleiter eine Untergrundmessung durchgeführt wird.

Durch die erfindungsgemäße Lehre wird die Möglichkeit geschaffen, Auswerteteil und Messteil voneinander zu trennen, da durch die erfindungsgemäße Verwendung von Lichtleitern eine Schnittstelle zur Verfügung gestellt wird, die weitgehend längenunabhängig ist.

Dies bedeutet, dass Messteil und Auswerteteil über Lichtleiter verbunden sein können, die in den einzelnen Baugruppen enden.

Des Weiteren wird erfindungsgemäß eine Messpunkteverteilung verwendet, die nicht zwingend mit den Mikrolinsen- bzw. Pinholeverteilung übereinstimmen muss, so dass infolgedessen eine einfache Auswertung möglich ist.

Wesentliche Elemente der erfindungsgemäßen Messanordnung sind folglich
- eine Beleuchtungseinheit, in der das Licht einer geeigneten Lichtquelle in die Lichtleiter eingekoppelt wird
- ein Element zur Trennung von Beleuchtungsstrahlengang und Detektionsstrahlengang
- ein Lichtleiterbündel mit einer geeigneten beleuchtungs- und detektionsseitigen räumlichen Anordnung der Lichtleiter und einer geeigneten probenseitigen Anordnung der Lichtleiter
- ein Objektiv mit starker chromatischer Aberration zur Abbildung der Lichtleiterenden auf das Messobjekt und zur Abbildung des vom Messkörper remittierten Lichts auf die Lichtleiterenden
- einer Farbmesseinheit zur Bestimmung der jeweiligen Peak-Lage und damit des Abstands des Messpunkts zum Objektiv.

Diese Farbmesseinheit besteht vorzugsweise aus einem dispersiven Element zur spektralen Aufspreizung des Lichts eines jeden Lichtleiters entlang einer Linie und einem Kamera-Chip, auf welchen die spektral aufgespreizten Messpunkte abgebildet werden. Die beleuchtungs- und detektionsseitige Anordnung der Lichtleiter ist so gewählt, dass einerseits eine möglichst effiziente Lichteinkopplung aus der Lichtquelle und andererseits eine möglichst effiziente räumliche Ausnutzung des Kamera-Chips gewährleistet ist. Aufgrund der erfindungsgemäßen Lehre besteht die Möglichkeit, eine große Anzahl von Lichtleitern zu verwenden und damit möglichst viele Messpunkte mit ausreichender spektraler Genauigkeit vermessen zu können.

Die Anordnung der Lichtleiter im Handstück und damit auf der Messkörperseite ist so gewählt, dass man, in Verbindung mit einer nachfolgenden Überlagerung von Einzelbildern, eine möglichst vorteilhafte Verteilung der Messpunkte auf dem Messkörper erhält.

Ist der Abstand der Stützstellen größer als die geforderte Auflösung, wird das Beleuchtungsmuster entsprechend verschoben werden. Dies kann entweder durch ein geeignetes Element im Gerät erfolgen, oder durch kontinuierliche Bewegung des Messgerätes, wobei die resultierenden Einzelbilder in geeigneter Weise zu einem Gesamtbild zusammenzufügen sind.

Insbesondere ist vorgesehen, dass das Licht auf die beleuchtungs- bzw. detektionsseitigen Enden der Lichtleiter linienförmig fokussiert wird. Hierdurch wird auch das remittierte Licht linienförmig auf die Detektiereinrichtung abgebildet mit der Folge, dass eine Auswertung nicht nur vereinfacht, sondern präzisiert wird.

Die linienförmige Fokussierung erfolgt insbesondere über Zylinderlinsen.

Als Lichtquellen werden insbesondere breitbandige Lichtquellen wie Halogenlampen, Xenonlampen und vor allem LEDs, entweder Weißlicht-LEDs oder RGD-LEDs verwendet. Dabei besteht die Möglichkeit, die Lichtleiter moduliert, gepulst oder geblitzt zu bestrahlen. Infolgedessen kann in Verbindung mit einer zur Lichtquelle synchronisierten Detektion eine effiziente Störlichtunterdrückung erreicht werden.

Ferner besteht die Möglichkeit, mehrere Lichtquellen zu verwenden, wodurch die Gesamtlichtmenge erhöht wird. Die räumliche Anordnung der einzelnen Lichtquellen kann dabei auf die räumliche Anordnung der Lichtleiter angepasst sein.

Bevorzugterweise wird als Detektiereinrichtung eine Farbkamera verwendet. Je nach Anforderung an die Messgenauigkeit ist entweder eine Ein-Chip-Farbkamera, eine Drei-Chip-Farbkamera oder eine Farbkamera mit Filterrad-Technologie zu verwenden. Nach einem eigenerfinderischen Vorschlag ist vorgesehen, dass die Lichtleiter beleuchtungs- bzw. detektionsseitig in Reihen bzw. Linien angeordnet sind. Die Lichtleiter werden sodann quasi verdrillt, so dass probenseitig eine gleichmäßige Verteilung der Lichtleiter erfolgt. Ungeachtet dessen ist eine korrekte Messung möglich, in dem durch Kalibrieren eine Zuordnung der proben seitigen Position der einzelnen Lichtleiter mit dem beleuchtungsseitigen Enden erfolgt.

Des Weiteren zeichnet sich die Erfindung dadurch aus, dass zur Verminderung des Untergrundanteils für eine Einzelmessung jeweils nur ein Teil wie die Hälfte der Lichtleiter für die eigentliche Messung, der andere Teil, der nicht beleuchtet wird, zur Untergrundmessung herangezogen wird. Die Variation der Beleuchtung kann beispielsweise entweder über einen LCD-Modulator oder einen variierenden Strahlversatz einer verkippbaren Glasplatte erfolgen. Die Lichtleiter einer Linie dienen bei einer Einzelmessung entweder jeweils alle zur Abstandsmessung oder zur Untergrundmessung.

Des Weiteren sieht die Erfindung vor, dass die Messanordnung eine das Objekt bestrahlende zweite Lichtquelle aufweist. Dabei kann der Spektralbereich der zweiten Lichtquelle außerhalb des Wellenlängenbereichs der zur Messung verwendeten Lichtquelle liegen. Hierdurch besteht die Möglichkeit, ein Live-Bild zu erzeugen.

Zur Erzeugung eines Live-Bilds ist auch die Integration eines Kamera-Chips mit einem Objektiv in Betracht zu ziehen. Entsprechende Bauelemente sind sodann in dem Teil der Messanordnung integriert, mit der gemessen wird, also üblicherweise einem Handgeräteteil, das über die Lichtleiter mit einer Auswerteeinheit verbunden ist.

Die Erfindung betrifft folglich eine Vorrichtung zur Vermessung der dreidimensionalen Form von Körpern mit insbesondere
a) einer Lichtquelle zur Erzeugung von Licht mit einem breitbandigen Spektrum
b) einer Optik zur Einkopplung des Lichts in ein Lichtleiterbündel
c) einem Lichtleiterbündel
d) einem Objektiv mit großer chromatischer Aberration zur Abbildung der Auskoppelenden der Lichtleiter auf den zu vermessenden Körper und zur rückwärtigen Abbildung des von dem Körper remittierten Lichts in die Auskoppelenden der Lichtleiter
e) einer Farbmesseinheit zur simultanen Aufnahme des Wellenlängenspektrums jeden Lichtleiters
f) einer Auswerteeinheit zur Bestimmung der spektralen Peaklage in jedem Fokus, aus der letztendlich der Abstand der jeweiligen Stelle zum Objektiv und damit die dreidimensionale Struktur des Körpers bestimmt wird.

Die Erfindung betrifft ferner ein mit einer solchen Vorrichtung durchführbares Messverfahren.

Weitere Einzelheiten, Vorteile und Merkmale der Erfindung ergeben sich nicht nur aus den Ansprüchen, den diesen zu entnehmenden Merkmalen -für sich und/oder in Kombination -, sondern auch aus der nachfolgenden Beschreibung von bevorzugten Ausführungsbeispielen und insbesondere den nachstehenden ergänzenden Erläuterungen.

Es zeigen:
- Abb. 1: eine Ausführungsform einer beleuchtungs- bzw. detektionsseitigen Anordnung von Lichtleitern und
- Abb. 2a, 2b: Anordnungen von Lichtleitern.

Nachstehend werden wesentliche Merkmale der Erfindung erläutert, um die Form eines Objekts zu messen. Dabei wird als Beispiel ein Zahn angegeben, ohne dass hierdurch die erfindungsgemäße Lehre eingeschränkt wird.

Unabhängig hiervon nimmt die Erfindung ausdrücklich auf die Offenbarung der Internationalen Anmeldung WO 2008/129073 (PCT/EP2008/054982) der Anmelderin Bezug. Die in dieser Anmeldung offenbarten Merkmale insbesondere in Bezug auf die Auswertung von Spektren und die Anordnung der zur Messung benötigten Komponenten sind als in der vorliegenden Anmeldung offenbart zu betrachten, ohne dass es nachstehend insoweit weiterer Hinweise bedarf.

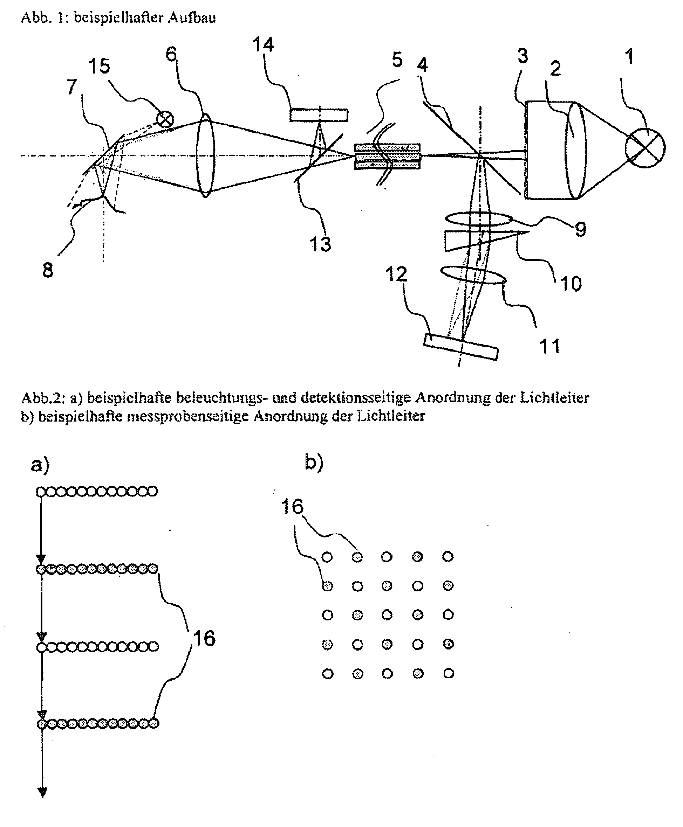

In Abb. 1 ist beispielhaft eine bevorzugte Ausführungsform zur Vermessung von Zähnen dargestellt. Als Weißlichtquelle dient hier eine Halogen-Lampe 1, deren Licht über eine Linse 2 kollimiert wird.

Der kollimierte Lichtstrahl fällt auf eine Anordnung von z.B. 10 Zylinderlinsen 3 und anschließend auf einen Strahlteiler 4. Der transmittierte Lichtanteil wird folglich zu zehn Linien fokussiert und wird dort beispielsweise in 2000 Lichtleiter 5 eingekoppelt, die zu je 200 Lichtleiter pro Linie angeordnet sind.

Die messkörperseitige Anordnung der Lichtleiter 5 bzw. der Teile dieser in einem Handstück entspricht beispielsweise einem regelmäßigen quadratischen Muster von 40 x 50 Messpunkten mit einem Messpunkteabstand von 220µm und einer Ausdehnung des Messfelds von ca. 8.6mm x 10.8mm.

Die Abbildung der Lichtleiterenden auf die Messprobe 8 erfolgt über das Objektiv 6 mit starker chromatischer Abhängigkeit der Brennweite und die Strahlumlenkung 7. Dabei befinden sich die Enden der Lichtleiter 5 in einer Abbildungsebene bzw. in Abbildungsebenen des Objektivs 6, die einen Bereich bilden.

Durch die starke chromatische Aberration des Objektivs 6 wird in dem jeweiligen Messpunkt, entsprechend dem Abstand von Messpunkt zu Objektiv 6, nur eine bestimmte Farbe scharf abgebildet, d.h. nur eine bestimmte Wellenlänge erfüllt die konfokale Bedingung. Mit anderen Worten weisen die Foki bildenden abgebildeten objektseitigen Enden der Lichtleiter 5 Licht unterschiedlicher Wellenlängen auf, wobei bei einer Wellenlänge das objektseitige Ende auf dem Objekt 6 scharf abgebildet ist und somit die konfokale Bedingung erfüllt. Entsprechend fällt bei der rückwärtigen Abbildung des von der Messprobenoberfläche remittierten Lichts nur der spektrale Anteil vorzugsweise in den Lichtleiter 5, der scharf abgebildet wird, d.h. bei dem die konfokale Bedingung erfüllt ist.

Der gewählte Abbildungsmaßstab des Objektivs 6 bestimmt dabei die Größe des Messfelds und die Auflösung.

Mit zunehmender Dichte der Messpunkte und mit zunehmender Lichtstreuung des Messobjekts 8 fällt neben der Peakwellenlänge, also der Wellenlänge, bei der das objektseitige Ende des Lichtleiters scharf abgebildet ist, auch ein zunehmender Störlichtanteil in die Lichtleiter 5. Mit dem dadurch zunehmenden Weißlichtanteil wird jedoch die Bestimmung der spektralen Peaklage erschwert, weshalb bereits ab 1% Weißlichtanteil keine sinnvolle Bestimmung der Peaklage mithilfe einer Farbkamera möglich ist. Deshalb wird erfindungsgemäß eine spektrometrische Anordnung gewählt, bei der das aus den Lichtleitern 5 austretende Licht über den Strahlteiler 4 und über Optiken 9 und 11 auf einem Kamerachip 12 abgebildet wird, während ein Prisma 10 eine spektrale Aufspreizung des Lichts bewirkt. Das Prisma 10 ist dabei zwischen den Optiken 9 und 11 angeordnet. Somit ergibt sich eine Anordnung wie diese der WO 2008/129073 zu entnehmen ist. Erwähntermaßen wird auf die diesbezügliche Offenbarung ausdrücklich Bezug genommen und gilt als in der vorliegenden Anmeldung offenbart.

Durch die entsprechende Anordnung wird ein Lichtleiterende jeweils auf eine Linie auf dem Kamerachip 12 abgebildet, wobei, wie bei einem gewöhnlichen Zeilen-Spektrometer, die Position entlang dieser Linie mit einer bestimmten Wellenlänge korreliert. Der Abstand zwischen den zehn Linien, bestehend jeweils aus 200 Lichtleitern 5, ist so gewählt, dass das am Strahlteiler 4 reflektierte und auf den Kamera-Chip 12 fallende Licht eines jeden Lichtleiters 5 überlappungsfrei bis zur nächsten Linie entlang von z.B. 100 Pixeln spektral zerlegt wird. Bei einem Kamera-Chip mit 1024 Pixel x 1024 Pixel stehen so für jeden Messpunkt, d.h. für jeden Lichtleiter, ein ca. 5 Pixel breiter und 100 Pixel langer Streifen für die spektrale Zerlegung zur Verfügung.

Nach Bildaufnahme erfolgt die Auswertung der Bildinformation bzw. der Messdaten entweder bereits auf dem Kamerachip 12 oder auf einer externen Einheit. Hierzu wird in jedem Messpunkt durch einen geeigneten Algorithmus die spektrale Peaklage und daraus der Abstand jedes Messpunkts zum Objektiv 6 ermittelt. Mit einem Bild erhält man so die dreidimensionale Struktur des Messobjekts 8 in den 2000 Stützstellen.

Ist der Abstand der Stützstellen größer als die geforderte Auflösung und/oder die dreidimensionale Struktur aus einer Perspektive nicht erfassbar, muss das Beleuchtungsmuster entsprechend verschoben werden. In der dargestellten Ausführungsform erfolgt dies durch eine Bewegung des Handstücks, wobei die resultierenden Einzelbilder in geeigneter Weise zu einem Gesamtbild zusammengefügt werden.

Zur Positionierhilfe und zur Einzelbildzuordnung für die Gesamtbild-Erzeugung ist in dem Ausführungsbeispiel ein weiterer Kamerachip 14 zur Aufnahme eines Livebilds vorgesehen. Für die Livebildaufnahme sind eine oder mehrere Lichtquellen 15 in einem Spektralbereich außerhalb des für die eigentliche Messung verwendeten Wellenlängenbereichs vorgesehen. In dem Ausführungsbeispiel sind die Lichtquellen 15 und der Kamerachip 14 im Handstück integriert. Die Strahlteilung erfolgt über einen geeigneten Strahlteiler 13. Die axiale Position des Kamerachips 14 ist so gewählt, dass das Livebild ungefähr in der Mitte des Messbereichs scharf ist.

Das angesprochene Handstück umfasst im Ausführungsbeispiel einen Abschnitt der Lichtleiter 5 sowie die objektseitig sodann angeordneten Bauelemente, d.h. Strahlteiler 13, Kamerachip 14, Objektiv 6, Zusatzlichtquelle 15 sowie Strahlenumlenkung 7. Die Auswerteeinheit umfasst die verbleibenden Teile. Handstück und Auswerteeinheit sind über Lichtleiter untereinander verbunden. Somit befinden sich die objektseitigen Enden, die in der bzw. den Abbildungsebenen des Objektivs 6 verlaufen, in dem Handgerät und die beleuchtungs- bzw. detektionsseitigen Enden in der getrennt hiervon angeordneten Auswerteeinheit.

Als breitbandige Lichtquellen kommen u. a. Halogenlampen, Xenonlampen und vor allem LEDs, entweder Weißlicht-LEDs oder RGB-LEDs in Frage. Die Verwendung von LEDs bietet die Möglichkeit einer modulierten bzw. einer gepulsten bzw. geblitzten Bestrahlung (mit höherer Leistung im Puls als im kontinuierlichen Betrieb). Dadurch kann in Verbindung mit einer zur Lichtquelle synchronisierten Detektion eine effiziente Störlichtunterdrückung erreicht werden.

Auch ist die Verwendung von mehreren Lichtquellen möglich. Bei LEDs kann dadurch die Gesamtlichtmenge erhöht werden. Die räumliche Anordnung der einzelnen Lichtquellen kann dabei auf die räumliche Anordnung der Lichtleiter angepasst sein. Die einzelnen Lichtquellen beleuchten damit jeweils nur einen bestimmten Anteil der Lichtleiter.

Vor allem bei Messaufgaben mit niedrigem Störlichtuntergrund ist als Farbmesseinheit die Verwendung einer Farbkamera möglich. Je nach Anforderung an die Messgenauigkeit ist entweder eine Ein-Chip-Farbkamera, eine Drei-Chip-Farbkamera oder eine Farbkamera mit Filterrad-Technologie möglich.

Je höher der Anteil des Störlichtuntergrunds am Messsignal ist, durch Streuung und/oder durch Störlicht von außen, umso wichtiger wird es, für eine exakte Bestimmung der Peaklage den Spektralverlauf des Untergrunds ebenfalls zu messen. Dies wird möglich, wenn ein Teil der Lichtleiter 5 nicht beleuchtet wird und damit probenseitig nur das remittierte Licht des Untergrundanteils erfasst wird.

In der in Abb. 2a dargestellten Ausführungsform liegen die Lichtleiter zur Untergrundbestimmung 16 beleuchtungs- bzw. detektionsseitig alle in einer Linie, die nicht beleuchtet wird. Probenseitig (Abb. 2b) sind die Lichtleiter 5 zur Untergrundbestimmung 16 gleichmäßig unter den anderen Lichtleitern verteilt.

Um den Untergrundanteil zu verringern, kann für eine Einzelmessung jeweils nur eine Hälfte der Lichtleiter 5 für die eigentliche Messung, der andere Teil, der nicht beleuchtet wird, für die Untergrundmessung verwendet werden. Die Variation der Beleuchtung kann beispielsweise entweder über einen LCD-Modulator oder den variierenden Strahlversatz einer verkippbaren Glasplatte erfolgen. Die Lichtleiter einer Linie dienen bei einer Einzelmessung entweder jeweils alle zur Abstandsmessung oder zur Untergrundmessung.

Eine Bestimmung des Untergrunds könnte, hier nur als Beispiel erwähnt, auch dadurch erfolgen, dass nach einer Messung die Bildebene durch ein optisches Element, beispielsweise durch Einschieben einer Glasplatte verschoben wird, und die Messung wiederholt wird. Dadurch verschiebt sich der Peak im Spektrum um einen definierten Wert in einen anderen Wellenlängenbereich. Durch geeignete Verrechnung der beiden Spektren lässt sich der Untergrund weitgehend eliminieren. Der gesuchte Abstand kann dabei aus der Peaklage beider Spektren bestimmt werden.

Neben der Verwendung einzelner Zylinderlinsen zur Beleuchtung der linienförmig angeordneten Lichtleiter ist die Verwendung eines speziell angepassten (Mikro-) Zylinderlinsen-Arrays möglich.

Werden zur Lichteinkopplung linienförmige Foki erzeugt, dann kann die Einkoppeleffizienz durch Verwendung von Lichtleitern ohne einkoppelseitigem Fasermantel gesteigert werden, da sich dann die Lichtleiter ohne Abstand nahe aneinander reihen lassen.

Zur Verbesserung der Einkoppeleffizienz ist aber auch die Kombination von Zylinderlinsen mit einem Mikrolinsen-Array oder die Verwendung von gekreuzten Zylinderlinsen möglich, um ein Muster aus einzelnen Foki zu erzeugen. Zu jedem Fokus muss dann entsprechend eine Faser positioniert sein.

Neben einer gleichmäßigen, quadratischen probenseitigen Anordnung der Lichtleiterenden sind auch andere Anordnungen denkbar. Zum Beispiel ist eine rotationssymmetrische Anordnung denkbar, die fertigungstechnische Vorteile hat. Denkbar ist auch eine unterschiedliche Dichte der Lichtleiter pro Fläche, um zum Beispiel den geringeren Überlappungsgrad der Einzelbilder im Randbereich durch eine höhere Dichte von Messpunkten auszugleichen. Alternativ ist eine im Randbereich abnehmende Messpunktedichte bei gleichzeitiger Vergrößerung des Messfelds möglich, um eine Verkippung zwischen versetzten Einzelbildern besser zu erkennen. Besonders einfach zu fertigen ist eine zufällige Anordnung der Lichtleiter, wobei der (mittlere) Abstand der Lichtleiter entweder durch den Fasermantel selbst oder durch zufällig beigemischte Blindfasern oder anderen (zylindrischen) Bauelementen eingestellt werden kann. Die Zuordnung der probenseitigen Position der einzelnen Lichtleiter zur zugehörigen Spektrum-Position auf dem Detektor erfolgt durch Kalibration an einem geeigneten Prüfkörper bekannter Geometrie.

Um eine fälschliche Lichtleitung im Fasermantel zu vermeiden, kann probenseitig und/oder einkoppelseitig anstelle des Mantels ein absorbierendes Material verwendet werden. Wenn die Anzahl der Lichtleiter von Standard-Faserbündeln nicht ausreichen, ist auch die kombinierte Verwendung von mehreren Standardfaserbündeln denkbar.

Ist der Durchmesser bzw. die Lichtmenge eines einzelnen Lichtleiters zu gering, ist die Verwendung mehrerer Lichtleiter pro Messstelle denkbar.

Auch ist eine funktionelle Aufteilung der Lichtleiter in Unterbündeln möglich. Dadurch kann z.B. das Licht der Lichtleiter, die nur zur Untergrundmessung verwendet werden, auf einen eigenen Kamera-Chip abgebildet werden, um beispielsweise die Kameraverstärkung getrennt für die eigentliche Abstandsmessung bzw. für die Untergrundmessung anpassen zu können. In Kombination mit mehreren Lichtquellen ist damit auch eine Aufteilung der Abstandsmessung in mehrere Spektralbereiche mit jeweils angepasster Lichtquelle (LED) und angepasster Kamera, gegebenenfalls mit angepasstem Farbfilter, möglich.

Um eine Verkleinerung der Durchmesser der Objektivlinsen und damit eine weitere Minimierung des Handstücks bei annähernd unveränderter Messfeldgröße zu erreichen, ist anstelle eines Objektivs mit telezentrischer Abbildung auch ein nicht telezentrisches Objektiv denkbar.

Besonders hilfreich für eine einfache Anpassung des Messgeräts an die jeweilige Fragestellung bzw. Messprobe ist die Realisierung eines Aufbaus mit wechselbarem chromatischem Objektiv. Je nach Abbildungsmaßstab, chromatischem Fehler und anderen optischen Kenngrößen lässt sich damit das Messgerät u. a. hinsichtlich Größe und Dichte der Messpunkte, Messabstand, Messbereich und Telezentrie an die Messaufgabe anpassen.

Ebenfalls für eine Anpassung an die jeweilige Messaufgabe können verschiedene Strahlumlenkungen, beispielsweise Umlenkspiegel oder Prismen unterschiedlicher Form und Funktion, verwendet werden. Diese sind probenseitig nach dem chromatischen Objektiv angeordnet. Es ist aber auch eine Strahlumlenkung innerhalb des Objektivs oder zwischen Faserenden und Objektiv möglich.

Für eine Referenzierung der Abstandsmessung kann ein definierter Rückreflex beispielsweise an der Auskoppelfläche des Umlenkprismas verwendet werden. Hierzu erfolgt beispielsweise vor jedem Messdurchgang eine Messung ohne Probe, wobei die jeweilige Peaklage in diesen Spektren dem Rückreflex zugeordnet werden kann. Bei der eigentlichen Messung an den Messproben kann es vorteilhaft sein, jeweils das Referenzspektrum vom Messspektrum abzuziehen.

Zur Erzeugung des Livebilds ist auch die Integration eines Kamerachips mit eigenem Objektiv im Handstück denkbar, ähnlich der "Chip on the Tip"-Technologie in der Endoskopie.

Um einen guten Überblick über die Messstelle zu haben, ist eine möglichst große Schärfentiefe für das Livebild wünschenswert. Diese kann verbessert werden, wenn in dem gemeinsamen optischen Strahlengang von Messung und Livebild, z.B. vorteilhaft in einer Pupillenebene des chromatischen Objektivs, eine dichroitische Blende eingesetzt wird, die nur für den Spektralbereich des Livebilds wirkt. Durch eine Verringerung der numerischen Apertur des Objektivs fürs Livebild kann so eine Erhöhung der Tiefenschärfe erzielt werden.

Wird durch eine große Schärfentiefe des Livebilds die Kontrolle des richtigen Messabstands zu sehr erschwert, ist als zusätzliche axiale Positionierhilfe die Einblendung einer Abstandsanzeige in das Livebild denkbar, wobei der Anzeigewert aus den 3D-Messdaten generiert werden kann.

Besonders, wenn nur wenige Lichtleiter eines oder mehrerer Faserbündel für die Messung verwendet werden, können die ungenutzten Lichtleiter für das Livebild verwendet werden.

Alternativ ist die Verwendung eines zusätzlichen Faserbündels für das Livebild möglich. Die messprobenseitige Zusammenführung des Mess-Strahlengangs und des Live-bild-Strahlengangs erfolgt dann über eine geeignete Optik.

Alternativ kann ohne eines zusätzlichen Kamera-Chips aus den Signalwerten des Messbilds und durch Kombination mehrerer Einzelbilder ein Livebild mit befriedigender Auflösung erzeugt werden. Dies erfolgt z.B. unter Verwendung der jeweiligen Peakhöhe in jedem Messpunktspektrum oder durch Integration aller Werte eines jeden Spektrums zu jeweils einem Helligkeitswert.

Alternativ wird detektionsseitig über einen weiteren Teilerspiegel ein Teil des Messlichts ohne spektrale Aufspaltung direkt auf einen weiteren Kamera-Chip abgebildet. Aus mehreren Einzelbildern kann dann durch geeignete Verrechnung ein Livebild mit höherer Auflösung generiert werden. Durch Verwendung der 3D-Daten kann die Verrechnung der Einzelbilder verbessert werden. Je nach Beschaffenheit des Messkörpers lässt sich die Qualität des Einzelbilds entweder durch Verwendung einer zusätzlichen, schmalbandigen Lichtquelle oder durch Verwendung nur eines Teils des Spektralbereichs des Messlichts für die Livebild-Detektion verbessern. Die Auswahl des Spektralbereichs kann über die Verwendung eines dichroitischen Teilerspiegels erfolgen.

## Patentansprüche

1. Verfahren zum Messen der Form zumindest eines Abschnitts eines Objekts (8), insbesondere eines semitransparenten Objekts wie zumindest Abschnitts eines Zahns, unter Verwendung zumindest einer Lichtquelle (1) zur Erzeugung von Licht mit vorzugsweise einem breitbandigen Spektrum, einer Einrichtung (3) zur Erzeugung eines multifokalen Beleuchtungsmusters, eines Objektivs (6) mit großer chromatischer Aberration zur Abbildung von Foki des Beleuchtungsmusters auf das Objekt, einer Detektiereinrichtung (12) zur Ermittlung der Wellenlängenspektren der konfokal über das Objektiv auf das Objekt abgebildeten Foki, wobei aus dem jeweiligen Wellenlängenspektrum spektrale Peaklage eines jeden Fokus bestimmt wird, aus der die Erstreckung des Objekts in Richtung des Abbildungsstrahls (Z-Koordinate) berechnet wird, das multifokale Beleuchtungsmuster über zwischen der Lichtquelle (1) und dem Objektiv (6) großer chromatischer Aberration angeordnete Lichtleiter (5) erzeugt wird, wobei das Objektiv (6) objektseitige Enden der Lichtleiter auf das Objekt und von dem Objekt remittiertes Licht auf die objektseitigen Enden der Lichtleiter abbildet und durch die Lichtleiter geleitetes remittiertes Licht auf die Detektiereinrichtung (12) gelenkt wird, wobei
eine unterschiedliche objektseitige Anordnung der Enden der Lichtleiter (5) und beleuchtungs- bzw. detektionsseitige Anordnung der Enden der Lichtleiter erzeugt wird, damit eine objektseitige Messpunkteverteilung von einer beleuchtungs- bzw. detektionsseitigen Mikrolinsen- bzw. Pinholeverteilung unabhängig ist,
**dadurch gekennzeichnet,**
**dass** ein Teil der Lichtleiter (5) unbeleuchtet bleibt und dass über den unbeleuchteten Teil der Lichtleiter eine Untergrundmessung durchgeführt wird.

2. Verfahren nach Anspruch 1,
**dadurch gekennzeichnet,**
**dass** eine beleuchtungs- bzw. detektionsseitige Anordnung der Lichtleiter derart gewählt wird, dass einerseits eine effiziente Lichteinkopplung aus der zumindest einen Lichtquelle (1) und andererseits eine effiziente räumliche Ausnutzung der Detektiereinrichtung (12) gewährleistet ist.

3. Verfahren nach Anspruch 1 oder 2,
**dadurch gekennzeichnet,**
**dass** objektseitig eine Anordnung der Lichtleiter (5) derart gewählt wird, dass in Verbindung mit einer nachfolgenden Überlagerung von Einzelbildern eine optimale Messpunkteverteilung auf dem Objekt (8) gewährleistet ist.

4. Verfahren nach zumindest einem der vorherigen Ansprüche,
**dadurch gekennzeichnet,**
**dass** das Beleuchtungsmuster objektseitig zur Aufnahme von Einzelbildern verschoben wird, wobei resultierende Einzelbilder zu einem Gesamtbild zusammengefügt werden.

5. Verfahren nach zumindest einem der vorherigen Ansprüche,
**dadurch gekennzeichnet,**
**dass** die Lichtleiter (5) beleuchtungs- bzw. detektionsseitig in Reihen bzw. Linien angeordnet werden, wobei die Lichtleiter vorzugsweise verdrillt werden, so dass probenseitig eine gleichmäßige Verteilung der Lichtleiter (5) erfolgt, wobei durch Kalibrieren eine Zuordnung der objekt- bzw. probenseitigen Position der einzelnen Lichtleiter (5) mit den beleuchtungsseitigen Enden der Lichtleiter erfolgt.

6. Verfahren nach zumindest einem der vorherigen Ansprüche,
**dadurch gekennzeichnet,**
das remittierte Licht eines jeden Lichtleiters (5) entlang einer Linie aufgespreizt wird, wobei das entlang einer Linie aufgespreizte Licht auf einen Kamera-Chip (12) geleitet wird.

7. Verfahren nach zumindest einem der vorherigen Ansprüche,
**dadurch gekennzeichnet,**
**dass** die Detektiereinrichtung eine die Wellenlängenspektren erfassende Pixelfläche eines Chips (12) wie CCD Sensors aufweist, dass die Pixelfläche und/oder die dispersive Einrichtung (9, 10, 11), die aus dem Lichtleiter (5) austretendes Licht lateral aufspreizt, zu den eine Ebene aufspannenden beleuchtungs- bzw. detektionsseitigem Enden der Lichtleiter (5) derart geneigt wird, dass die aus den Lichtleitern austretende Strahlung überlappungsfrei auf der Pixelfläche auftrifft.

8. Verfahren nach zumindest einem der vorherigen Ansprüche,
**dadurch gekennzeichnet,**
**dass** das Licht auf die beleuchtungs- bzw. detektionsseitigen Enden der Lichtleiter (5) vorzugsweise über Zylinderlinsen (3) linienförmig fokussiert wird.

9. Verfahren nach zumindest einem der vorherigen Ansprüche,
**dadurch gekennzeichnet,**
**dass** von einem eine konfokale Bedingung erfüllenden und einen Lichtleiter (5) durchsetzenden Strahlenbündel ein erstes Spektrum ermittelt wird, dass in dem Strahlengang zwischen dem Objekt (8) und dem Lichtleiter ein den Strahlengang änderndes optisches Element angeordnet wird, dass ein zweites Spektrum von dem Strahlenbündel mit geändertem Strahlengang ermittelt wird und dass die Spektren voneinander subtrahiert und aus sich ergebenden gleichen Peaks unterschiedlichen Vorzeichens Wellenlänge des Strahlenbündels ermittelt wird.

10. Messanordnung zum dreidimensionalen Messen von zumindest einem Teil eines Objekts, insbesondere eines semitransparenten Objekts (8) wie Zahns oder Abschnitts eines solchen, umfassend zumindest eine Lichtquelle (1) mit einem kontinuierlichen, insbesondere breitbandigen Spektrum, eine Einrichtung (3, 5) zur Erzeugung eines multifokalen Beleuchtungsmusters, ein Objektiv (6) mit großer chromatischer Abberation zur Abbildung von Foki des Beleuchtungsmusters auf das Objekt, eine Detektiereinheit (12) wie Kamera-Chip zur Ermittlung der Wellenlängenspektren der über das Objektiv auf das Objekt abgebildeten Foki sowie eine spektraldispersive Einrichtung (9, 10, 11), auf die von dem Objekt remittiertes Licht über das Objektiv abbildbar ist, wobei zwischen der Lichtquelle (1) und dem Objektiv (6) das Beleuchtungsmuster erzeugende Lichtleiter (5) angeordnet sind, deren objektseitige Enden in einer Abbildungsebene oder einem Abbildungsebenen umfassenden Bereich des Objektivs angeordnet sind, und zwischen den beleuchtungsseitigen Enden der Lichtleiter und der Detektiereinrichtung (12) eine Umlenkeinrichtung (4) für das aus dem Lichtleiter austretende und von dem Objekt remittierte Licht angeordnet ist, wobei
die objektseitigen Enden der Lichtleiter (5) derart angeordnet sind, dass objektseitig eine Messpunkteverteilung abbildbar ist, die von einer beleuchtungs- bzw. detektionsseitigen Mikrolinsen- bzw. Pinholeverteilung unabhängig ist,
**dadurch gekennzeichnet,**
**dass** ein Teil der Lichtleiter (5) unbeleuchtet ist und dass dadurch zur Untergrundmessung probenseitig nur das remittierte Licht des Untergrundanteils erfasst wird, das über den Teil der unbeleuchteten Lichtleiter auf die Detektiereinheit (12) oder einen gesonderten Kamera-Chip abbildbar ist..

11. Messanordnung nach Anspruch 10,
**dadurch gekennzeichnet,**
**dass** die Messanordnung mehrere Lichtquellen (1) umfasst, wobei eine räumliche Anordnung der einzelnen Lichtquellen (1) auf die räumliche Anordnung der Lichtleiter (5; 16) angepasst ist.

12. Messanordnung nach zumindest Anspruch 10,
**dadurch gekennzeichnet,**
**dass** die Lichtleiter (5) beleuchtungs- bzw. detektionsseitig in Reihen bzw. in Linien angeordnet sind und/oder dass die Lichtleiter (5) derart ausgebildet, vorzugsweise verdrillt sind, dass die Lichtleiter probenseitig eine gleichmäßige Verteilung aufweisen, wobei insbesondere die Lichtleiter (5) zu einem Bündel zusammengefasst sind, das aus Unterbündeln besteht, wobei über ein Unterbündel die Untergrundmessung durchführbar ist.

13. Messanordnung nach zumindest Anspruch 10,
**dadurch gekennzeichnet,**
**dass** die Lichtleiter zur Untergrundbestimmung (16) beleuchtungs- bzw. detektionsseitig alle in einer Linie liegen, die nicht beleuchtet ist und dass die Lichtleiter (5) zur Untergrundbestimmung (16) probenseitig gleichmäßig unter den anderen Lichtleitern verteilt sind.

14. Messanordnung nach zumindest einem der Ansprüche 10 bis 13,
**dadurch gekennzeichnet,**
**dass** die Lichtleiterenden probenseitig gleichmäßig, quadratisch und/oder rotationssymmetrisch angeordnet sind und/oder dass die Lichtleiter pro Fläche eine unterschiedliche Dichte aufweisen und/oder in einem Randbereich eine abnehmende Messpunktedichte bei gleichzeitiger Vergrößerung eines Messfeldes vorgesehen ist und/oder dass die Lichtleiter (5) zufällig angeordnet sind, wobei ein (mittlerer) Abstand der Lichtleiter (5) entweder durch einen Fasermantel selbst oder durch zufällig beigemischte Blindfasem oder andere (zylindrische) Bauelemente einstellbar ist.

15. Messanordnung nach zumindest Anspruch 10,
**dadurch gekennzeichnet,**
**dass** das Licht über Zylinderlinsen (3) oder linienförmig fokussiertes Licht auf die beleuchtungsseitigen Enden der Lichtleiter (5) abbildbar ist, die vorzugsweise einkoppelseitig fasermantelfrei sind.

16. Messanordnung nach zumindest Anspruch 10,
**dadurch gekennzeichnet,**
**dass** die Messanordnung aus einem Handteil und einem beabstandet zu diesem anordbaren Messteil besteht, die über die Lichtleiter (5) verbunden sind, wobei insbesondere das Handteil die objektseitigen Enden der Lichtleiter (5), das Objektiv (6) mit großer chromatischer Aberration sowie zumindest eine Umlenkeinrichtung (7) umfasst und/oder die Messanordnung ein wechselbares chromatisches Objektv (6) aufweist, das vorzugsweise telezentrisch abbildet.

17. Messanordnung nach zumindest Anspruch 10,
**dadurch gekennzeichnet,**
**dass** die Lichtleiterenden zumindest objektseitig als Bündel mit vorzugsweise quadratischem Querschnitt zusammengefasst sind, wobei insbesondere die Lichtleiter (5) zu einem Bündel zusammengefasst sind, das aus Unterbündeln besteht, wobei über ein Unterbündel die Untergrundmessung durchfuhrbar ist.

18. Messanordnung nach zumindest Anspruch 10,
**dadurch gekennzeichnet,**
**dass** die Messanordnung, insbesondere das Handgerät, einen Kamerachip (14) zur Erzeugung eines Live-Bilds aufweist.

19. Messanordnung nach zumindest einem der Ansprüche 10 bis 18,
**dadurch gekennzeichnet,**
**dass** die Lichtquelle (1) eine Halogenlampe, eine Xenonlampe oder ein oder mehrere LEDs, insbesondere Weißlicht-LEDs oder RGB-LEDs, sind, wobei vorzugsweise die LEDs modulierte oder gepulste Strahlung emittieren oder die LEDs die Leiterenden blitzartig bestrahlen.

20. Messanordnung nach zumindest Anspruch 10,
**dadurch gekennzeichnet,**
**dass** die Detektionseinrichtung (12) eine 1-Chip-Farbkamera, eine 3-Chip-Farbkamera oder eine Farbkamera mit Filterrad-Technologie umfasst.

## Claims

1. A method for measuring the shape of at least a section of an object (8), in particular of a semi-transparent object such as at least a section of a tooth, using at least one light source (1) to generate light with preferably a broad-band spectrum, a device (3) to generate a multi-focal illumination pattern, an objective lens (6) with high chromatic aberration to image foci of the illumination pattern onto the object, a detector unit (12) to determine the wavelength spectra of the foci imaged confocally onto the object by the objective lens, whereby the spectral peak position of each focus is determined from the corresponding wavelength spectrum, from which the extension of the object towards the imaging beam (z coordinate) is computed, whereby the multi-focal illumination pattern is generated by light guides (5) that are arranged between the light source (1) and the objective lens (6) with high chromatic aberration, whereby the object lens (6) images ends of the light guides on the object side onto the object and images light remitted by the object onto the ends of the light guides on the object side, and whereby remitted light that is conducted through the light guides is directed onto the detector device (12), whereby a differing arrangement of the ends of the light guides (5) on the object side and arrangement of the ends of the light guides on the illumination side, respectively detection side is generated, so that a measuring point distribution on the object side is independent of a distribution of microlenses, respectively pinholes on the illumination side, respectively detection side,
**characterized in**
**that** a part of the light guides (5) remains unlighted and that a background measurement is performed via the unlighted part of the light guides.

2. The method according to claim 1,
**characterized in**
**that** an arrangement of the light guides on the illumination or detection side is chosen to ensure an efficient coupling-in of light from the at least one light source (1) on the one hand and an efficient spatial utilization of the detector device (12) on the other.

3. The method according to claim 1 or 2,
**characterized in**
**that** an arrangement of the light guides (5) on the object side is chosen such that in combination with a subsequent superposition of individual images an optimum distribution of measuring points on the object (8) is ensured.

4. The method according to at least one of the preceding claims,
**characterized in**
**that** the illumination pattern on the object side is shifted for the recording of individual images, whereby resulting individual images are merged into an overall image.

5. The method according to at least one of the preceding claims,
**characterized in**
**that** the light guides (5) on the illumination or detection side are arranged in rows, respectively lines, whereby the light guides preferably are twisted in order to obtain a uniform distribution of the light guides (5) on the sample side, whereby calibration is used to assign the positions of the individual light guides (5) on the object or sample side to the ends of the light guides on the illumination side.

6. The method according to at least one of the preceding claims,
**characterized in**
**that** the remitted light of each light guide (5) is spread along a line, whereby the light spread along a line is directed onto a camera chip (12).

7. The method according to at least one of the preceding claims,
**characterized in**
**that** the detector device comprises a pixel surface of a chip (12), such as a CCD sensor, which detects the wavelength spectra, that the pixel surface and/or the dispersive device (9, 10, 11), that laterally spreads light exiting from the light guide 5, are inclined relative to the plane defined by the ends of the light guides (5) on the illumination or detection side in such a way that the radiation emerging from the light guides impinges upon the pixel surface without overlap.

8. The method according to at least one of the preceding claims,
**characterized in**
**that** the light is focused in lines, preferably through cylinder lenses (3), on the ends of the light guides (5) on the illumination or detection side.

9. The method according to at least one of the preceding claims,
**characterized in**
**that** a first spectrum is determined by a bundle of rays that satisfies a confocal condition and passes through a light guide (5), that in the beam path between the object (8) and the light guide is arranged an optical element that modifies the beam path, that a second spectrum is determined by the bundle of rays with modified beam path, and that the spectra are subtracted from each other, and that wavelength of the bundle of rays is determined from resulting identical peaks with opposite sign.

10. A measuring arrangement for the three-dimensional measuring of at least a part of an object, in particular of a semi-transparent object (8), such as a tooth or section thereof, comprising at least a light source (1) with a continuous in particular wide-band spectrum, a device (3, 5) to generate a multi-focal illumination pattern, an objective lens (6) with high chromatic aberration to image foci of the illumination pattern onto the object, a detector unit (12), such as a camera chip, to determine the wavelength spectra of the foci imaged onto the object by the objective lens, as well as a spectrum-dispersing device (9, 10, 11), onto which light returning remitted by the object can be imaged, whereby arranged between the light source (1) and the objective lens (6) are light guides (5), which generate the illumination pattern and whose ends on the object side are arranged in an imaging plane or an imaging planes comprising region of the objective lens, and between the ends of the light guides on the illumination side and the detector device (12) is arranged a deviating device (4) for the light emerging from the light guide and remitted by the object, whereby the ends of the light guides (5) on the object side are arranged in a manner so that a measuring point distribution can be imaged on the object side that is independent of a distribution of microlenses or pinholes on the illumination or detection side,
**characterized in**
**that** a part of the light guides (5) is unlighted and that for background measuring thereby is determined at the sample side only the remitted light of the background portion, that can be imaged on the detector device (12) or a separate camera chip through the portion of the unlighted light guides.

11. The measuring arrangement according to claim 10,
**characterized in**
**that** the measuring arrangement comprises several light sources (1), whereby a spatial arrangement of the individual light sources (1) is adapted to the spatial arrangement of the light guides (5; 16).

12. The measuring arrangement according to at least claim 10,
**characterized in**
**that** the light guides (5) on the illumination or detection side are arranged in rows or lines and/or that the light guides (5) are designed, preferably twisted, such that the light guides on the sample side exhibit a uniform distribution, whereby especially the light guides (5) are combined into a bundle that consists of sub-bundles, whereby the background measurement can be performed through a sub-bundle.

13. The measuring arrangement according to at least claim 10,
**characterized in**
**that** the light guides for background determination (16) on the illumination or detection side are all arranged along one line that is not illuminated, and that the light guides (5) for background determination (16) on the sample side are distributed uniformly among the other light guides.

14. The measuring arrangement according to at least one of claims 10 to 13,
**characterized in**
**that** the light guide ends on the sample side are arranged uniformly, in a square pattern and/or rotationally symmetric pattern, and/or that the light guides per surface area exhibit a varying density, and/or that a decreasing measuring point density is intended in a border region, with a simultaneous increase of a measuring field and/or that the light guides (5) are arranged randomly, whereby a (mean) distance of the light guides (5) can be set either using a fibre sheath itself or using randomly admixed blind fibres or other (cylindrical) components.

15. The measuring arrangement according to at least claim 10,
**characterized in**
**that** through cylindrical lenses (3) or light focused in lines the light can be imaged on the ends of the light guides (5) on the illumination side which preferably on the coupling-in side are free of a fibre sheath.

16. The measuring arrangement according to at least claim 10,
**characterized in**
**that** the measuring arrangement consists of a hand-set and a measuring unit located at a distance thereto, which are connected through the light guides (5), whereby especially the hand-set comprises the ends of the light guides (5) on the object side, the objective lens (6) with high chromatic aberration, as well as at least one deviating device (7) and/or that the measuring arrangement comprises an interchangeable chromatic objective lens (6), which preferably (6) images telecentrically.

17. The measuring arrangement according to at least claim 10,
**characterized in**
**that**, at least on the object side, the light guide ends are combined into a bundle with preferably a square cross section, whereby especially the light guides (5) are combined into a bundle that consists of sub-bundles, whereby the background measurement can be performed through a sub-bundle.

18. The measuring arrangement according to at least claim 10,
**characterized in**
**that** the measuring arrangement, in particular the hand-set, comprises a camera chip (14) to generate a live image.

19. The measuring arrangement according to at least one of claims 10 to 18,
**characterized in**
**that** the light source (1) is a halogen lamp, a xenon lamp, or one or several LEDs, especially white-light LEDs or RGB LEDs, whereby preferably the LEDs emit modulated or pulsed radiation or the LEDs illuminate the light guide ends in a flashed mode.

20. The measuring arrangement according to at least claim 10,
**characterized in**
**that** the detector device (12) comprises a 1-chip colour camera, a 3-chip colour camera, or a colour camera with filter-wheel technology.

## Revendications

1. Procédé destiné à mesurer la forme d'au moins une partie d'un objet (8), en particulier d'un objet semi-transparent tel qu'au moins une partie d'une dent, en utilisant au moins une source lumineuse (1) pour générer une lumière avec de préférence un spectre à large bande, un dispositif (3) pour générer un modèle d'éclairage multifocal, un objectif (6) à forte aberration chromatique pour reproduire sur l'objet des foyers du modèle d'éclairage, un dispositif détecteur (12) pour déterminer les spectres de longueurs d'ondes des foyers reproduits de manière confocale par le biais de l'objectif sur l'objet, sachant qu'à partir de chaque spectre de longueurs d'ondes est déterminée la pointe spectrale de chaque foyer, à partir de laquelle est calculée l'extension de l'objet dans la direction du rayon de reproduction (Z), que le modèle d'éclairage multifocal est généré par des conducteurs optiques (5) à forte aberration chromatique placés entre la source lumineuse (1) et l'objectif (6), que l'objectif (6) reproduit sur l'objet les extrémités côté objet des conducteurs optiques et la lumière renvoyée par l'objet sur les extrémités côté objet des conducteurs optiques, que la lumière renvoyée conduite par les conducteurs optiques est dirigée sur le dispositif détecteur (12), et
que sont créées une configuration côté objet des extrémités des conducteurs optiques (5) et une configuration côté éclairage et/ou détection des extrémités des conducteurs optiques différentes, de sorte qu'une répartition des points de mesure est indépendante d'une répartition côté éclairage et/ou détection de microlentilles et/ou de piqûres,
**caractérisé en ce**
**qu'**une partie des conducteurs optiques (5) reste non éclairée et qu'est effectuée une mesure de fond par le biais de la partie non éclairée des conducteurs optiques.

2. Procédé selon la revendication 1,
**caractérisé en ce**
**qu'**une configuration côté éclairage et/ou détection des conducteurs optiques est choisie de manière telle que sont assurées d'une part une injection efficace de la lumière issue de l'au moins une source lumineuse (1) et d'autre part une exploitation efficace dans l'espace du dispositif détecteur (12).

3. Procédé selon la revendication 1 ou 2,
**caractérisé en ce**
**qu'**une configuration côté objet des conducteurs optiques (5) est choisie de manière telle, qu'en liaison avec une superposition consécutive d'images isolées, est assurée une répartition optimale des points de mesure sur l'objet (8).

4. Procédé selon au moins une des revendications précédentes,
**caractérisé en ce**
**que** le modèle d'éclairage côté objet est déplacé pour enregistrer des images isolées, les images isolées résultantes étant réunies en une image globale.

5. Procédé selon au moins une des revendications précédentes,
**caractérisé en ce**
**que** les conducteurs optiques (5) côté éclairage et/ou détection sont disposés en rangées et/ou en lignes, sachant que de préférence les conducteurs optiques sont torsadés de sorte qu'est obtenue côté échantillon une répartition régulière des conducteurs optiques (5), sachant que par calibrage est obtenue une correspondance de la position côté objet et/ou échantillon des différents conducteurs optiques (5) avec les extrémités côté éclairage des conducteurs optiques.

6. Procédé selon au moins une des revendications précédentes,
**caractérisé en ce**
**que** la lumière renvoyée par chaque conducteur optique (5) est écartée/déviée le long d'une ligne, la lumière écartée/déviée le long d'une ligne étant dirigée sur une puce caméra (12).

7. Procédé selon au moins une des revendications précédentes,
**caractérisé en ce**
**que** le dispositif détecteur présente une surface de pixels d'une puce (12), telle qu'un capteur CDD, saisissant les spectres de longueurs d'ondes, que la surface de pixels et/ou le dispositif dispersif (9, 10, 11) qui écarte/dévie latéralement la lumière sortant du conducteur optique (5), est/sont incliné(e)(s) par rapport aux extrémités côté éclairage et/ou détection des conducteurs optiques (5) décrivant un plan, de manière telle que le rayonnement sortant des conducteurs optiques frappe la surface de pixels sans chevauchement.

8. Procédé selon au moins une des revendications précédentes,
**caractérisé en ce**
**que** la lumière aux extrémités côté éclairage et/ou détection des conducteurs optiques (5) est de préférence linéairement focalisée par des lentilles cylindriques (3).

9. Procédé selon au moins une des revendications précédentes,
**caractérisé en ce**
**qu'**est déterminé un premier spectre d'un faisceau de rayons remplissant un condition confocale et traversant un conducteur optique (5), que sur la trajectoire du faisceau entre l'objet (8) et le conducteur optique est placé un élément optique modifiant la trajectoire du faisceau, qu'est déterminé un second spectre du faisceau de rayons avec trajectoire modifiée, que les spectres sont soustraits l'un de l'autre et que la longueur d'onde du faisceau de rayons est déterminée à partir des pointes égales de signes différents qui en résultent.

10. Système de mesure destiné à la mesure tridimensionnelle d'au moins une partie d'un objet, en particulier d'un objet semi-transparent (8), tel que d'une dent ou d'un segment de dent, comprenant au moins une source lumineuse (1) avec un spectre continu, en particulier à large bande, un dispositif (3, 5) pour générer un modèle d'éclairage multifocal, un objectif (6) à forte aberration chromatique pour reproduire sur l'objet des foyers du modèle d'éclairage, une unité de détection (12) telle qu'une puce caméra pour déterminer les spectres de longueurs d'ondes des foyers reproduits sur l'objet par le biais de l'objectif, ainsi qu'un dispositif de dispersion spectrale (9, 10, 11) sur lequel la lumière renvoyée par l'objet peut être reproduite par le biais de l'objectif, sachant qu'entre la source lumineuse (1) et l'objectif (6) sont placés des conducteurs optiques (5) qui génèrent le modèle d'éclairage et dont les extrémités côté objet sont disposées dans un plan de reproduction ou dans une zone comprenant un plan de reproduction de l'objectif, et qu'entre les extrémités côté éclairage des conducteurs optiques et le dispositif détecteur (12) est placé un dispositif déviateur (4) pour la lumière sortant des conducteurs optiques et renvoyée par l'objet, et
que les extrémités côté objet des conducteurs optiques (5) sont disposées de manière telle que du côté objet peut être reproduite une répartition des points de mesure qui est indépendante d'une répartition côté éclairage et/ou détection de microlentilles et/ou de piqûres,
**caractérisé en ce**
**qu'**une partie des conducteurs optiques (5) est non éclairée et qu'ainsi pour la mesure de fond côté échantillon, seule est saisie la lumière renvoyée par la part de fond, qui peut être reproduite par la partie des conducteurs optiques non éclairée sur l'unité de détection (12) ou une puce caméra distincte.

11. Système de mesure selon la revendication 10,
**caractérisé en ce**
**que** le système de mesure comprend plusieurs sources lumineuses (1), une configuration dans l'espace des différentes sources lumineuses (1) étant adaptée à la configuration dans l'espace des conducteurs optiques (5 ; 16).

12. Système de mesure selon au moins la revendication 10,
**caractérisé en ce**
**que** les conducteurs optiques (5) côté éclairage et/ou détection sont disposés en rangées et/ou en lignes, et/ou que les conducteurs optiques (5) sont conçus, de préférence torsadés, de sorte que les conducteurs optiques présentent une répartition régulière côté échantillon, sachant qu'en particulier les conducteurs optiques (5) sont réunis en un faisceau constitué de sous-faisceaux, la mesure de fond pouvant être effectuée par le biais d'un sous-faisceau.

13. Système de mesure selon au moins la revendication 10,
**caractérisé en ce**
**que** les conducteurs optiques pour la détermination du fond (16) côté éclairage et/ou détection sont tous placés dans une ligne qui n'est pas éclairée et que les conducteurs optiques (5) pour la détermination du fond (16) côté échantillon sont régulièrement réparties parmi les autres conducteurs optiques.

14. Système de mesure selon au moins une des revendications 10 à 13,
**caractérisé en ce**
**que** les extrémités des conducteurs optiques côté échantillon sont régulièrement réparties en carrés et/ou selon une symétrie de révolution et/ou que les conducteurs optiques présentent une densité différente par unité de surface et/ou que dans une zone marginale est prévue une densité de points de mesure décroissante pour un grossissement simultané d'un champ de mesure et/ou que les conducteurs optiques (5) sont disposés au hasard, sachant qu'un espacement (moyen) des conducteurs optiques (5) est réglable soit au moyen d'une gaine de fibre elle-même, soit au moyen de fibres aveugles ou autres composants (cylindriques) ajouté(e)s au hasard.

15. Système de mesure selon au moins la revendication 10,
**caractérisé en ce**
**qu'**au moyen de lentilles cylindriques (3) ou d'une lumière linéairement focalisée, la lumière peut être reproduite sur les extrémités côté éclairage des conducteurs optiques (5), qui sont de préférence sans gaine de fibre du côté couplage.

16. Système de mesure selon au moins la revendication 10,
**caractérisé en ce**
**que** le système de mesure est constitué d'une partie manuelle et d'une partie de mesure qui peuvent être placées à distance l'une de l'autre et sont reliées par les conducteurs optiques (5), sachant qu'en particulier la partie manuelle comprend les extrémités côté objet des conducteurs optiques (5), l'objectif (6) à forte aberration chromatique ainsi qu'au moins un dispositif déviateur (7) et/ou que le système de mesure présente un objectif chromatique interchangeable (6) qui de préférence reproduit de manière télécentrique.

17. Système de mesure selon au moins la revendication 10,
**caractérisé en ce**
**qu'**au moins côté objet, les extrémités des conducteurs optiques sont réunies en faisceau avec une coupe transversale de préférence carrée, sachant qu'en particulier les conducteurs optiques (5) sont réunis en un faisceau constitué de sous-faisceaux, la mesure de fond pouvant être effectuée par le biais d'un sous-faisceau.

18. Système de mesure selon au moins la revendication 10,
**caractérisé en ce**
**que** le système de mesure, en particulier l'appareil manuel, présente une puce caméra (14) pour générer une image en direct.

19. Système de mesure selon au moins une des revendications 10 à 18,
**caractérisé en ce**
**que** la source lumineuse (1) est une lampe à halogène, une lampe à xénon, ou une ou plusieurs LED, en particulier LED à lumière blanche ou LED RVB, sachant que de préférence les LED émettent un rayonnement modulé ou pulsé, ou que les LED irradient les extrémités des conducteurs en lançant des éclairs.

20. Système de mesure selon au moins la revendication 10,
**caractérisé en ce**
**que** le dispositif de détection (12) comprend une caméra couleur à 1 puce, une caméra couleur à 3 puces ou une caméra couleur dotée de la technologie des roues filtrantes.
